# EUROPÄISCHE PATENTSCHRIFT

(11) **EP 3 481 298 B1**
(45) Veröffentlichungstag und Bekanntmachung des Hinweises auf die Patenterteilung: **18.09.2019**
(21) Anmeldenummer: 17793853.7
(22) Anmeldetag: 13.10.2017
(51) Int. Cl.: A61B 5/113, A61B 5/18, A61B 5/00, B60K 28/02

(54) **VERFAHREN ZUM BETREIBEN EINES KRAFTFAHRZEUGS**
METHOD FOR OPERATING A MOTOR VEHICLE
PROCÉDÉ POUR FAIRE FONCTIONNER UN VÉHICULE AUTOMOBILE

(30) Priorität: 30.01.2017 DE 102017201405
(43) Veröffentlichungstag der Anmeldung: 15.05.2019
(73) Patentinhaber: Audi AG, 85045 Ingolstadt (DE)
(72) Erfinder: STARK, Christiane, 85123 Karlskron (DE); BORGERT, Isabelle, 85053 Ingolstadt (DE)
(86) Internationale Anmeldenummer: PCT/EP2017/076204
(87) Internationale Veröffentlichungsnummer: WO 2018/137799

(56) Entgegenhaltungen:
- WO-A1-2015/127193
- WO-A2-2015/200224
- JP-A- 2009 213 636
- JP-A- 2016 220 816
- US-A1- 2004 044 293
- US-A1- 2014 276 090

## Beschreibung

Die Erfindung betrifft ein Verfahren zum Betreiben eines Kraftfahrzeugs und ein Kraftfahrzeug. Das Kraftfahrzeug weist eine Sensoreinrichtung zum Erfassen mindestens eines eine Oberkörperbewegung eines Benutzers des Kraftfahrzeugs beschreibenden Atmungsparameters und zum Erfassen einer Bewegung eines Kraftfahrzeugsitzes des Kraftfahrzeugs beschreibenden Fahrbewegungsparameters auf.

In modernen Kraftfahrzeugen sind Kontrollsysteme zum Verbessern einer Fahrsicherheit vorgesehen. Solche Kontrollsysteme können physiologische Parameter des Benutzers, sogenannte Vitalparameter, während der Fahrt ermitteln und/oder erfassen und daraus zum Beispiel eine Fahrtüchtigkeit des Benutzers ermitteln.

Die DE 10 2015 002 968 A1 berücksichtigt beispielsweise eine Fahrtstrecke oder eine Uhrzeit oder einen Kraftstoffverbrauch bei der Beurteilung des Fahrerzustandes.

Die DE 10 2009 053 407 A1 schlägt zuvor, zum Beispiel eine Frequenz eines Herzschlages oder eine Atemfrequenz des Fahrzeuginsassen zu erfassen. Gemäß der WO 2015/140273 A2 wird ein Beschleunigungssensor in den Sicherheitsgurt integriert, um auf die Herztätigkeit oder die Atmung und schießen zu können.

Die DE 10 2004 016 191 A1 beschreibt hierzu eine Technologie, bei der zum Erfassen der Atemfrequenz ein Bewegungssensor in einem Haltegurt integriert ist und eine Bewegung eines Brustkorbs des Benutzers oder des Bauches erfasst. Bei der Signalauswertung wird so die Atemfrequenz und/oder Herzfrequenz herausgefiltert und abgeleitet.

Das Erfassen einer Atembewegung des Benutzers mithilfe einer am Sicherheitsgurt angeordneten Sensoreinrichtung wird ebenfalls von der DE 10 2005 059 687 A1 vorgeschlagen, wobei ein zugempfindliches Element eine Erweiterung des Brustkorbs misst. Dabei ist eine Korrektureinheit vorgesehen, die eine Fahrzeugbewegung bei der Ermittlung der Atemfrequenz berücksichtigt.

Die US 2004/0044293 A1 beschreibt ein System zum Überwachen einer Aufmerksamkeit oder Wachheit eines Benutzers eines Kraftfahrzeugs anhand von einem Wert, der eine Atmung beschreibt.

Das Erfassen von Vitalparametern zum Beispiel während einer Fahrt hat zunächst die Anwendung, dem Fahrer lediglich eine Information über seinen Zustand zukommen zu lassen. Andere Systeme beziehen einen medizinischen Aspekt mit ein und überwachen die Vitalparameter des Benutzers, um zum Beispiel in Notsituationen einen Notruf auszulösen. In neuerdings entwickelten Kraftfahrzeugen spielt jedoch ein neuer Aspekt eine Rolle, denn moderne Kraftfahrzeuge sind häufig mit Fahrerassistenzsystemen ausgestattet, die in vollautonomen Fahrmodi betrieben werden können. In solchen pilotiert fahrenden Kraftfahrzeugen kann es vorkommen, dass das Kraftfahrzeug die Steuerung an den Benutzer abgibt. Da der Benutzer während des vollautonomen oder pilotierten Fahrmodus sich jedoch anderen Tätigkeiten widmen kann, kann es Situationen geben, in denen einerseits das Kraftfahrzeug eine Übergabe der Steuerung auf den Benutzer vorbereitet, andererseits jedoch der Benutzer in diesem Moment zum Beispiel nicht sehr aufmerksam ist oder in diesem Moment die Steuerung besser nicht übernehmen sollte oder könnte.

Eine der Erfindung zu Grunde liegende Aufgabe ist das situationsabhängige Optimieren einer Übergabe einer Steuerung eines Kraftfahrzeugs.

Die Aufgabe wird von dem erfindungsgemäßen Verfahren und den erfindungsgemäßen Gegenständen der nebengeordneten Patentansprüche gelöst. Weitere vorteilhafte Ausführungsformen sind durch die Unteransprüche gegeben.

Die Erfindung basiert auf der Idee, benutzerspezifisch zu ermitteln, ob eine aktuelle Situation ein günstiger Moment für die Fahrzeugübergabe ist. Hierzu wird nicht nur ein reiner Zahlenwert des Vitalparameters berücksichtigt, sondem es wird überprüft, ob der aktuelle Wert des Parameters ein Wert ist, der für den individuellen Benutzer in einem normalen Wertebereich liegt oder zum Beispiel für diese Person einen kritischen Wert darstellt.

Das erfindungsgemäße Verfahren zum Betreiben eines Kraftfahrzeugs weist die folgenden Verfahrensschritte auf.

Durch eine Sensoreinrichtung des Kraftfahrzeugs erfolgen ein Erfassen mindestens eines eine Oberkörperbewegung eines Benutzers des Kraftfahrzeugs beschreibenden Atmungsbewegungsparameters und ein Erzeugen eines den erfassten Atmungsbewegungsparameter beschreibenden Atmungsbewegungssignals. Durch die Sensoreinrichtung erfolgen ebenfalls ein Erfassen eines eine Bewegung eines Kraftfahrzeugsitzes des Kraftfahrzeugs beschreibenden Fahrbewegungsparameters und ein Erzeugen eines den erfassten Fahrbewegungsparameter beschreibenden Fahrbewegungssignals. Als Sensoreinrichtung wird dabei ein Gerät oder eine Gerätekomponente verstanden, das oder die für die entsprechenden Verfahrensschritte eingerichtet ist und hierzu beispielsweise einen oder mehrere Sensoren aufweist. Zum Erfassen der Oberkörperbewegung kann beispielsweise ein Bewegungssensor oder ein Beschleunigungssensor in einem Anschnallgurt des Kraftfahrzeugs angeordnet sein. Zum Erfassen der Bewegung des Kraftfahrzeugsitzes kann beispielsweise ein weiterer Bewegungssensor oder ein weiterer Beschleunigungssensor in einem Sitzpolster des Kraftfahrzeugsitzes angeordnet sein.

Durch eine Auswerte- und Steuereinrichtung, also ein Gerät oder eine Gerätekomponente zur elektronischen Datenverarbeitung und zum Erzeugen von Steuersignalen, erfolgt anhand des Atmungsbewegungssignals und des Fahrbewegungssignals ein Ermitteln eines Vitalparameters, also eines physiologischen Parameters, der eine Atmung des Benutzers beschreibt. Der Vitalparameter kann dabei beispielsweise eine Atemtiefe und/oder eine Atemfrequenz sein. Die Auswerte- und Steuereinrichtung kann beispielsweise als Steuergerät oder Steuerplatine ausgestaltet sein.

Zum Ermitteln des Vitalparameters wird ein durch das Atmungsbewegungssignal beschriebener Wert anhand eines Wertes des Fahrbewegungssignals normalisiert, indem beispielsweise anhand des Fahrbewegungssignals ein Einfluss der Bewegung des Kraftfahrzeugsitz ist auf den erfassten Atmungsbewegungsparameter und/oder die erst Sensoreinrichtung ermittelt wird.

Anhand des ermittelten Einflusses kann dann zum Beispiel der Wert des Atmungsbewegungsparameters angepasst werden. Mit anderen Worten wird eine Oberkörperbewegung, die nicht durch die eigentliche Atmung sondern durch eine Bewegung des Fahrzeugs, bzw. des Kraftfahrzeugsitzes erfolgt, herausgefiltert oder herausgerechnet werden, sodass der Wert des Vitalparameters nur die tatsächlich durch die Atmung des Benutzers durchgeführte Bewegung beschreibt.

Gemäß dem erfindungsgemäßen Verfahren erfolgt ein Feststellen eines Referenzwertebereiches, in dem ein ermittelter Wert des Vitalparameters liegt. Der Referenzwertebereich umfasst dabei in einer Speichereinrichtung gespeicherte, benutzerspezifische Referenzwerte des Vitalparameters. Als Speichereinrichtung wird ein Bauteil oder eine Gerätekomponente verstanden, die zur elektronischen Datenspeicherung ausgelegt ist und beispielsweise als Festplatte oder Speicherkarte ausgestaltet sein kann. Beispielsweise können in der Speichereinrichtung auch mehrere Referenzwertebereiche gespeichert sein, von denen dann ein Referenzwertebereich ausgewählt werden kann.

Anhand des festgestellten Referenzwertebereichs erfolgt ein Feststellen eines Fahrerzustandsindex, der einen aktuellen physiologischen Zustand des Benutzers beschreibt. Mit anderen Worten kann beispielsweise in einer elektronisch abgespeicherten Liste dem Referenzwertebereich oder jedem von mehreren Referenzwertebereich ein unterschiedlicher Fahrerzustandsindex zugeordnet sein, der beispielsweise einen Grad einer Aufmerksamkeit oder Müdigkeit oder einen Grad eines Stresszustandes des Benutzers beschreiben kann.

Durch die Auswerte- und Steuereinrichtung erfolgt, in Abhängigkeit von dem ermittelten Fahrerzustandsindex, ein Erzeugen eines Steuersignals zum Betreiben einer Fahrerassistenzeinrichtung des Kraftfahrzeugs, und ein Übertragen des Steuersignals an die Fahrerassistenzeinrichtung.

Die Fahrerassistenzeinrichtung kann dann zum Beispiel einen aktuell eingestellten Fahrmodus in Abhängigkeit von dem ermittelten Fahrerzustandsindex abbrechen oder fortführen. Die Fahrerassistenzeinrichtung ist dabei ein Gerät oder ein Bauteil, das dazu eingerichtet und ausgelegt ist, das Kraftfahrzeug in einem oder mehreren Fahrmodi zum Betreiben, und kann beispielsweise ein gängiges Fahrerassistenzsystem des Kraftfahrzeugs sein.

Durch das erfindungsgemäße Verfahren werden die oben genannten Nachteile reduziert oder sogar behoben. Der die Atmung betreffende Vitalparameter eignet sich besonders gut für die Bewertung des Fahrerzustandes, da der Fahrerzustand sehr zuverlässig beschrieben werden kann. Dadurch, dass erst durch Vergleich mit dem benutzerspezifischen Referenzwertebereich der Fahrerzustandsindex ermittelt wird, kann zwischen unterschiedlichen Benutzern, die beispielsweise in unterschiedlicher körperlicher oder sportlicher Verfassung sein können, unterschieden werden. Deswegen kann eine zuverlässige Entscheidung getroffen werden, ob beispielsweise der Fahrer im Moment das Kraftfahrzeug zuverlässig und schnell übernehmen kann.

Beispielsweise hat ein Leistungssportler in der Regel ein sehr hohes Lungenvolumen und deswegen eine langsamere Atmung als ein untrainierter Benutzer des Kraftfahrzeugs. Ein Wert beispielsweise einer Atemtiefe, der bei einem Leistungssportler in einem Normbereich liegen und für einen normalen, entspannten aber wachen und konzentrierten Körperzustand sprechen kann, kann bei einem Benutzer, der selten Sport macht, ein Anzeichen für eine hohe Müdigkeit sein oder dafür, dass der Benutzer unter Umständen gerade schläft.

Wird beispielsweise die Atemtiefe als Vitalparameter gemessen, also eine Ausdehnung des Brustkorbes und/oder des Bauchs des Benutzers, so kann durch den Abgleich mit dem Referenzwertebereich eine Aussage über ein Lungenvolumen getroffen werden, also zum Beispiel wie viel Sauerstoff von dem Benutzer bei einem Atemzug aufgenommen werden kann. Ein flaches, schnelles Atmen kann bei einem ersten Benutzer beispielsweise ein normaler Zustand sein, während bei einem anderen Benutzer dies bereits ein Anzeichen von Stress sein kann. Mithilfe des erfindungsgemäßen Verfahrens können solche personenspezifische Unterschiede jedoch erkannt werden.

Durch das erfindungsgemäße Verfahren werden die Parameter in identischer Weise aufgenommen, d.h. zeitgleich und mit verwandten Technologien. Dadurch ist das Ermitteln des Vitalparameters besonders zuverlässig. Das erfindungsgemäße Verfahren ermöglicht eine umfassende Fahrzustandsanalyse durch beispielsweise Identifizieren von Atemmustern.

Gemäß einer besonders bevorzugten Ausführungsform des erfindungsgemäßen Verfahrens erfolgt durch die Auswerte- und Steuereinrichtung ein Feststellen einer Identität des Benutzers, beispielsweise anhand von Zugangsdaten, die der Benutzer beim Starten des Kraftfahrzeugs angeben kann, oder beispielsweise durch Erfassen eines biometrischen Merkmals des Benutzers oder eines Identifikationscodes zum Beispiel von einem Kraftfahrzeugschlüssel. Das Bereitstellen der benutzerspezifischen Referenzwerte des Vitalparameters und das Feststellen des Referenzwertebereichs erfolgt in Abhängigkeit von der festgestellten Identität. Das erfindungsgemäße Verfahren kann so für unterschiedliche Benutzer angewendet werden und dadurch wird das Betreiben des Kraftfahrzeugs noch besser personalisiert und sehr flexibel.

Es kann ein Empfangen mindestens eines Vitalparametersignals vorgesehen sein, das einen Wert des Vitalparameters oder eines weiteren Vitalparameters beschreiben kann, beispielsweise ein Wert, der einen Puls des Benutzers beschreiben kann. Vorzugsweise kann das Vitalparametersignal aus einem tragbaren Accessoire und/oder aus einem mobilen Endgerät empfangen werden. Das Feststellen des Fahrerzustandsindex kann dann in Abhängigkeit von dem empfangenen Vitalparametersignal erfolgen. Durch diese Variante des erfindungsgemäßen Verfahrens können zusätzlich zum Beispiel historische Vitalparameter genutzt werden, die der Benutzer beispielsweise außerhalb des Kraftfahrzeugs gesammelt hat, und die Aussage über den Fahrerzustand kann so noch besser verfeinert werden.

Den gleichen Vorteil bietet eine weitere Ausführungsform des erfindungsgemäßen Verfahrens, das durch ein Empfangen mindestens eines Betriebssignals aus einem Kraftfahrzeugsystem gekennzeichnet ist, das einen Betriebszustand des Kraftfahrzeugsystems beschreiben kann. Das Feststellen des Fahrerzustandsindex erfolgt dann in Abhängigkeit von dem empfangenen Betriebssignal. Mit anderen Worten können Sensorinformationen aus der Fahrzeugsensorik herangezogen werden, und daraus kann ebenfalls auf den Fahrerzustand rückgeschlossen werden. Beispielsweise kann so ein Einstellen einer Klimaanlage auf eine ungewöhnlich hohe Temperatur einen Hinweis darauf geben, dass der Benutzer zum Beispiel gestresst oder krank sein kann.

Um den Benutzer besser "kennenzulernen", also um das erfindungsgemäße Verfahren mit der Zeit immer weiter zu verfeinern und zu personalisieren, kann ein Speichern des ermittelten Werts des Vitalparameters als Referenzwert in der Speichereinrichtung vorgesehen sein, und ein Anpassen des Referenzwertbereichs in Abhängigkeit von dem gespeicherten Wert. Diese optionalen Verfahrensschritte können ebenfalls durch die Auswerte- und Steuereinrichtung durchgeführt werden.

Die oben gestellte Aufgabe wird ebenfalls gelöst durch eine Auswerte- und Steuereinrichtung, die vorzugsweise einen Mikrokontroller und/oder einen Mikroprozessor aufweisen kann, wobei die Auswerte- und Steuereinrichtung dazu eingerichtet ist, die eine Auswerte- und Steuereinrichtung betreffenden Verfahrensschritte gemäß einem Verfahren nach einer der oben beschriebenen Ausführungsformen durchzuführen. Es ergeben sich die oben genannten Vorteile.

Die Aufgabe wird, unter Erreichen der oben beschriebenen Vorteile, ebenfalls gelöst durch ein Kraftfahrzeug, aufweisend eine Fahrerassistenzeinrichtung und eine Sensoreinrichtung, wobei die Sensoreinrichtung einen Sensor zum Erfassen des die Oberkörperbewegung des Benutzers des Kraftfahrzeugs beschreibenden Atmungsbewegungsparameters aufweist und einen Sensor zum Erfassen des die Bewegung des Kraftfahrzeugs ist es beschreibenden Fahrbewegungsparameters. Das erfindungsgemäße Kraftfahrzeug ist durch eine Ausführungsform der erfindungsgemäßen Auswerte- und Steuereinrichtung gekennzeichnet, und kann beispielsweise als Kraftwagen, insbesondere als Personenkraftwagen, ausgestaltet sein.

Vorzugsweise kann dabei der Sensor zum Erfassen des Atmungsbewegungsparameters an einem Sicherheitsgurt des Kraftfahrzeugs angeordnet sein, und der Sensor zum Erfassen des Fahrbewegungsparameters kann an einem Kraftfahrzeugsitz angeordnet sein. Hierdurch können die entsprechenden Parameter besonders gut erfasst werden.

Die Vorteile werden ebenfalls erreicht durch eine Speichereinrichtung mit einem Programmcode, der dazu eingerichtet ist, bei Ausführen durch eine Auswerte- und Steuereinrichtung eines mobilen Endgeräts ein Verfahren nach einem der oben beschriebenen Ausführungsformen durchzuführen.

Die Erfindung betrifft ebenfalls ein mobiles, portables Endgerät mit einer erfindungsgemäßen Speichereinrichtung und eine Servervorrichtung zum Betreiben im Internet, aufweisend eine erfindungsgemäße Speichereinrichtung.

Im Folgenden sind Ausführungsbeispiele der Erfindung beschrieben. Hierzu zeigt:
Fig. 1 eine schematische Darstellung zu einem ersten Ausführungsbeispiel des erfindungsgemäßen Verfahrens;
Fig. 2 eine schematische Darstellung zu einem weiteren Ausführungsbeispiel des erfindungsgemäßen Verfahrens; und
Fig. 3 eine schematische Darstellung zu einem weiteren Ausführungsbeispiel des erfindungsgemäßen Verfahrens.

Bei den im Folgenden erläuterten Ausführungsbeispielen handelt es sich um bevorzugte Ausführungsformen der Erfindung. Bei den Ausführungsbeispielen stellen die beschriebenen Komponenten der Ausführungsformen jeweils einzelne, unabhängig voneinander zu betrachtende Merkmale der Erfindung dar, welche die Erfindung jeweils auch unabhängig voneinander weiterbilden und damit auch einzeln oder in einer anderen als der gezeigten Kombination als Bestandteil der Erfindung anzusehen sind. Des Weiteren sind die beschriebenen Ausführungsformen auch durch weitere der bereits beschriebenen Merkmale der Erfindung ergänzbar.

In den Figuren sind funktionsgleiche Elemente jeweils mit denselben Bezugszeichen versehen.

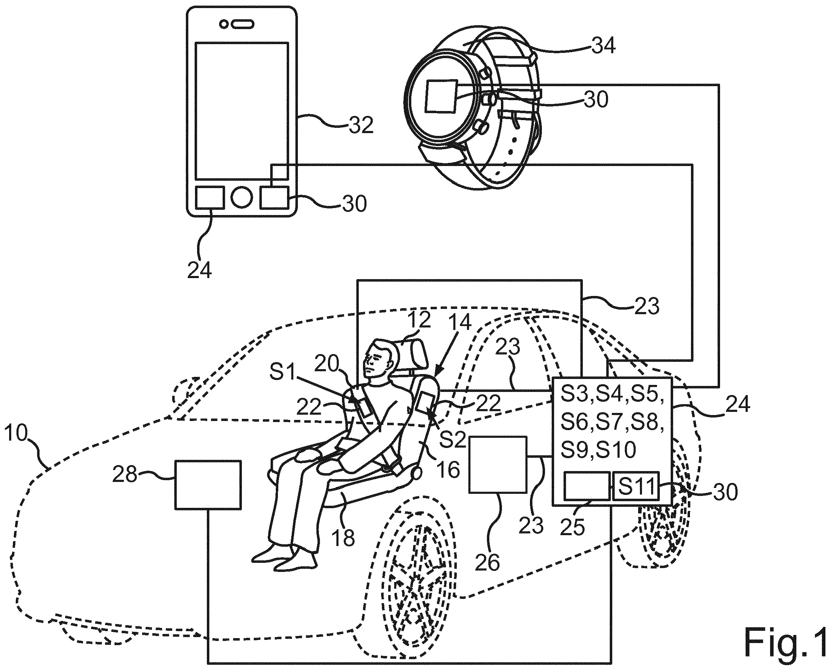

Die Fig. 1 beschreibt das Prinzip des erfindungsgemäßen Verfahrens anhand eines ersten Ausführungsbeispiels. Hierzu zeigt die Fig. 1 ein Kraftfahrzeug 10 eines Benutzers 12. Das Kraftfahrzeug 10 kann beispielsweise als Personenkraftwagen ausgestaltet sein. Der Benutzer 12 sitzt auf einem Kraftfahrzeugsitz 14 mit einer Rückenlehne 16, einem Sitzpolster 18 und einem Sicherheitsgurt 20.

Das Kraftfahrzeug 10 weist ebenfalls eine Sensoreinrichtung 22 auf, die beispielhaft zwei Sensoren aufweisen kann, wobei einer der Sensoren an den Sicherheitsgurt 20 angeordnet sein kann, und der andere der Sensoren an den Kraftfahrzeugsitz 14. Der Sensor zum Erfassen des Atmungsbewegungsparameters kann beispielsweise auf den Sicherheitsgurt 20 aufgeklebt oder in den Sicherheitsgurt 20 eingenäht sein. Der Sensor zum Erfassen des Fahrbewegungsparameters kann beispielhaft in die Rückenlehne 16 integriert sein, oder in das Sitzpolster 18. Hierbei sind jedoch auch alternative Varianten denkbar, beispielsweise mehrere Sensoren sowohl in der Rückenlehne 16, als auch in dem Sitzpolster 18.

Der Sensor zum Erfassen des Atmungsbewegungsparameters kann beispielsweise ein aus dem Stand der Technik bekannter Sensor zum Erfassen einer Beschleunigung und/oder einer Bewegung und/oder einer Erschütterung sein. Ebenso kann der Sensor zum Erfassen des Fahrbewegungsparameters zum Beispiel ein Bewegungs- und/oder Beschleunigungssensor sein. Der Sensor zum Erfassen des Fahrbewegungsparameters kann beispielsweise eine Eigenvibration des Kraftfahrzeugsitzes 14 erfassen.

Der Sensor zum Erfassen des Atmungsbewegungsparameters kann beispielsweise eine dreidimensionale Bewegung erfassen zum Messen einer Atemtiefe und/oder eine Atembewegung. Je nach Anwendung kann dieser Sensor an einer geeigneten Stelle des Sicherheitsgurts 20 angeordnet sein, beispielsweise an einer Stelle, wo der Sicherheitsgurt 20 eine Brust Mittelpunkt des Benutzers 12 aufliegt oder auf den Bauch des Benutzers 12. Da die Bewegungen des Kraftfahrzeugsitzes 14 auf den Körper des Benutzers 12 einwirken, werden von dem ersten Sensor nicht nur die durch Atmung bedingten Bewegungen des Oberkörpers erfasst, sondern die Gesamtbewegung des Oberkörpers.

Die Sensoreinrichtung 22 kann über eine Datenkommunikationsverbindung 23 mit einer Auswerte- und Steuereinrichtung 24 verbunden sein. Eine solche Datenkommunikationsverbindung 23 kann beispielsweise eine drahtgebundene Datenkommunikationsverbindung sein, beispielsweise ein Bestandteil eines Datenbussystems des Kraftfahrzeugs 10, oder eine drahtlose Datenkommunikationsverbindung, wie beispielsweise eine WLAN- oder Bluetooth LE Verbindung.

Die Auswerte- und Steuereinrichtung 24 kann beispielsweise als Steuergerät des Kraftfahrzeugs 10 ausgestaltet sein, als Softwaremodul oder als Steuerplatine. Die Auswerte- und Steuereinrichtung 24 kann optional einen Mikrokontroller 25 und/oder einen Mikroprozessor 25 aufweisen. Die Auswerteeinrichtung kann eine Speichereinrichtung 30 aufweisen, die beispielsweise als Speichermedium ausgestaltet sein kann, vorzugsweise als Datenspeicher und/oder Festplatte. Die Speichereinrichtung 30, sowie der Mikrokontroller 25 bzw. der Mikroprozessor 25, können untereinander kommunizieren. Optional können der Mikroprozessor 25 oder der Mikrokontroller 25 Zugriff auf einen in der Speichereinrichtung 30 gespeicherten Programmcode haben, wobei der Programmcode dazu eingerichtet ist, bei Ausführen durch die Auswerte- und Steuereinrichtung eine Ausführungsform des erfindungsgemäßen Verfahrens durchzuführen.

Das Kraftfahrzeug 10 weist ebenfalls eine Fahrerassistenzeinrichtung 26 auf, die beispielsweise als ein dem Fachmann auf dem Stand der Technik geläufige Fahrerassistenzsystem ausgestaltet sein kann, das zum Beispiel zum Betreiben des Kraftfahrzeugs 10 in einem pilotierten Fahrmodus eingerichtet sein kann.

Die Fig. 1 zeigt ebenfalls ein Kraftfahrzeugsystem 28 des Kraftfahrzeugs 10, dass beispielsweise als Klimaanlage oder Lenksystem oder Infotainmentsystem ausgestaltet sein kann.

Das Beispiel der Fig. 1 zeigt ebenfalls ein portables, mobiles Endgerät 32, das beispielsweise als Smartphone ausgestaltet sein kann. Hierbei zeigt Fig. 1, dass die Auswerte- und Steuereinrichtung 24 nicht im Kraftfahrzeug 10 angeordnet sein muss, sondern alternativ auch Bestandteil des mobilen, portablen Endgeräts 32 sein kann. Die entsprechenden Datenkommunikationsverbindungen, die vorzugsweise drahtlose Datenkommunikationsverbindung sein können, sind jedoch für diese Variante - aus Gründen der Übersichtlichkeit - nicht in der Fig. 1 gezeigt.

Schließlich zeigt die Fig. 1 ein tragbares Accessoire 34, dass beispielsweise als Armbanduhr oder Fitnessgurt ausgestaltet sein kann und auch als "Wearable" bezeichnet werden kann.

In einem ersten Verfahrensschritt S1 erfasst die Sensoreinrichtung 22 den Atmungsbewegungsparameter und erzeugt ein entsprechendes Sensorsignal, das den erfassten Atmungsparameter beschreibt. Beispielsweise erfasst die Sensoreinrichtung 22 eine 3-dimensionale Bewegung oder einen Druck. Gleichzeitig kann der Sensor der Sensoreinrichtung 22 eine Erschütterung oder eine Eigenvibration des Kraftfahrzeugsitzes 14 erfassen und ein entsprechendes Fahrbewegungssignals erzeugen (S2), das diese Eigenbewegung oder Erschütterung beschreibt. Die einzelnen Sensoren übertragen das jeweilige Signal an die Auswerte- und Steuereinrichtung 24.

Da sich eine Erschütterung des Kraftfahrzeugsitzes und beispielsweise ein gleichzeitiges Ausdehnen das Brustkorbes addieren und somit das Atmungsbewegungssignals die Summe aller Einwirkungen auf den Sensor in den Sicherheitsgurt 20 beschreibt, ermittelt die Auswerte- und Steuereinrichtung 24 im Verfahrensschritt S3 einen normalisierten Wert der Atembewegung, beispielsweise die tatsächliche Atemtiefe und/oder die tatsächlich nur durch den Brustkorb verursachte Atembewegung. Dazu kann beispielsweise der Wert des Fahrbewegungssignals von dem Wert des Atmungsbewegungsparameters abgezogen werden.

In der Speichereinrichtung 30 können beispielsweise eine Vielzahl von Referenzwerten des Benutzers 12 gespeichert sein, die beispielsweise in mehrere Referenzwertebereiche eingeteilt sein können. Dabei kann jeder der Referenzwertebereiche zum Beispiel eine Stufe des Vitalparameters zugeordnet sein, d.h. einem vorbestimmten Fahrerzustand. Im Beispiel der Fig. 1 kann das Kraftfahrzeug 10 beispielsweise regelmäßig von unterschiedlichen Benutzern 12 genutzt werden, beispielsweise von einem Leistungssportler und einem Benutzer 12, der eine normal übliche Kondition hat. Um individueller auf den aktuellen Benutzer 12 eingehen zu können, kann gemäß dem Verfahren vorgesehen sein, dass beispielsweise beim Öffnen des Kraftfahrzeugs 10 oder beim Starten des Kraftfahrzeugs 10 ein Identifikationsvorgang durchgeführt wird, bei dem eine Identität des Benutzers 12 festgestellt wird (S4).

Zum Feststellen der Identität (S4) kann beispielsweise ein Identifikationscode eines Kraftfahrzeugschlüssel abgefragt werden oder beispielsweise eine PIN, die der Benutzer 12 vor dem Start des Kraftfahrzeugs 10 eingegeben haben kann. Stellt die Auswerte- und Steuereinrichtung 24 beispielsweise fest (S4) dass der Leistungssportler nun das Kraftfahrzeug 10 benutzt, können diejenigen Referenzwerte des Vitalparameters bereitgestellt werden (S5) die zum Beispiel zu seinem Benutzerprofil gespeichert sind. Entsprechend der festgestellten Identität kann auch die Einordnung in Referenzwertebereiche erfolgen, oder die entsprechenden Referenzwertebereiche können festgestellt werden (S6). Dabei kann die Einteilung der Referenzwerte in die Referenzwertebereiche bereits benutzerspezifisch vorgegeben sein.

Beispielsweise kann der ermittelte Vitalparameter eine sehr langsame Atmung beschreiben. Während ein entsprechender Wert des Vitalparameters für den normal trainierten Benutzer 12 ein Anzeichen für zum Beispiel Müdigkeit sein kann, können die Referenzwerte des Leistungssportlers jedoch zum Beispiel beschreiben, dass dies ein normaler Wert für diesen Benutzer 12 in einem aufmerksamen Wachzustand ist. Im Verfahrensschritt S7 kann also festgestellt werden, dass der Benutzer 12 im Moment wachsam ist.

In der beispielhaften Situation der Fig. 1 kann sich das Kraftfahrzeug 10 beispielsweise einem Streckenabschnitt nähern, in dem pilotiertes Fahren nicht erlaubt ist. Zum Beispiel anhand von Kartendaten kann die Fahrerassistenzeinrichtung 26 erkennen, dass es vorher ratsam ist, die Steuerung des Kraftfahrzeugs 10 an den Benutzer 12 zu übergeben. Die Auswerte- und Steuereinrichtung 24 kann, da der Benutzer 12 Moment wach und aufmerksam ist, ein Steuersignal erzeugen (S8), das eine solche Übergabe und damit einen Abbruch des pilotierten Fahrmodus beschreiben kann. Im Verfahrensschritt S9 wird das Steuersignal dann an die Fahrerassistenzeinrichtung 26 übertragen. Die Fahrerassistenzeinrichtung 26 kann daraufhin beispielsweise den Benutzer 12 über den kommenden Kommandowechsel informieren und dann den pilotierten Fahrmodus ändern. Wäre alternativ der andere Benutzer 12 im Kraftfahrzeug 10, dessen Fahrerzustandsindex zum Beispiel beschreiben kann, dass er im Moment mit hoher Wahrscheinlichkeit sehr müde und deswegen weniger aufmerksam ist, kann es beispielsweise angebracht sein, noch im pilotierten Fahrmodus das Kraftfahrzeug 10 abzubremsen und beispielsweise an einem Seitenrand abzustellen, und erst dann den pilotierten Fahrmodus zu beenden.

Die Fig. 1 zeigt schematisch weitere Varianten, mit der das erfindungsgemäße Verfahren verfeinert werden kann. Beispielsweise kann der ermittelte Vitalparameter als Referenzwert aufgenommen werden. Alternativ oder zusätzlich kann ein Vitalparameter, auch ein Vitalparameter anderer Art, wie beispielsweise ein Puls oder ein Vitalparameter in Bezug auf eine Herztätigkeit, zum Beispiel aus der Speichereinrichtung 30 des tragbaren Accessoires 34 und/oder aus der Speichereinrichtung 30 des mobilen Endgeräts 32 empfangen werden (S10). Diese Daten können bei dem Vorgang des Feststellens des Fahrerzustandsindex (S7) mit einbezogen werden. Alternativ oder zusätzlich können diese Vitalparameter als Referenzwert in der Speichereinrichtung 30 der Auswerte- und Steuereinrichtung 24 des Kraftfahrzeugs 10 gespeichert werden (S11). Mit zunehmender Anzahl von Referenzwerten kann so eine Normbandbreite festgelegt werden, was zu einem treffsichereren Erkennen des aktuellen Zustands des Benutzers führt.

Zusätzlich oder alternativ können beispielsweise Betriebssignale aus dem Kraftfahrzeugsystem 28 oder mehreren Kraftfahrzeugsystemen 28 ausgewertet werden, die ebenfalls durch die Auswerte- und Steuereinrichtung 24 empfangen werden (S10). Hierbei kann also zum Beispiel das Verhalten des Benutzers 12 Kraftfahrzeug 10 analysiert werden.

Optional kann vorgesehen sein, dass der Fahrerzustandsindex zum Beispiel an ein Komfortsystem 28 übertragen werden kann, oder dass die Auswerte-und Steuereinrichtung 24 ein Steuersignal zum Betreiben des Komfortsystems 28 erzeugen und dieses an das Komfortsystem 28 übertragen kann. So kann beispielsweise, entsprechend dem Zustand des Fahrers, die Klimaanlage reguliert werden, oder es kann der Benutzer 12 zunächst ein Vorschlag gemacht werden, ob er beispielsweise eine Temperatur senken oder zum Beispiel eine Innenbeleuchtung ändern möchte.

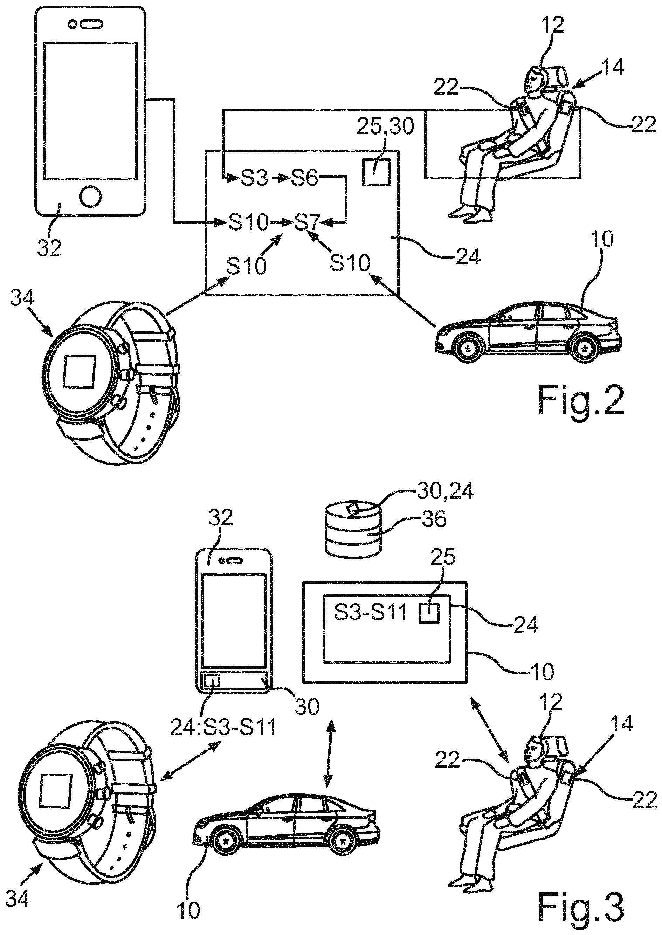

Die Fig. 2 zeigt schematisch ein weiteres Ausführungsbeispiel des erfindungsgemäßen Verfahrens, wobei im Folgenden nur noch auf die Unterschiede zu dem Verfahren der Fig. 1 eingegangen wird.

Die Fig. 2 veranschaulicht dabei noch einmal die möglichen Daten, mit deren Hilfe der Fahrerzustandsindex festgestellt werden kann (S7). Zusätzlich zu den Daten der Sensoreinrichtung 22 können Fahrzeugdaten des Kraftfahrzeugs 10 mit in den Fahrerzustandsindex einfließen, beispielsweise Daten zu einer Lenkbewegung. Optional können ebenfalls weitere Werte zu dem Vitalparameter oder Werte des anderen Vitalparameters in den Fahrerzustandsindex mit einfließen, beispielsweise jährliche und/oder aktuelle Vitalwerte aus einem tragbaren Accessoires 34. Alternativ oder zusätzlich ist jedoch auch denkbar, dass solche anderen Werte von Vitalparametern durch das Kraftfahrzeug 10 erfasst werden, beispielsweise mithilfe eines Pulssensors am Lenkrad. Aus dem mobilen Endgeräts 32 können optional Vitalparameter empfangen werden (S10), vorzugsweise sogenannte "geteilte Werte" eines Vitalparameters oder "geteilte Vitalwerte", also zum Beispiel historische Daten, die zum Beispiel von einem Anwenderprogramm des mobilen Endgeräts 32 über einen längeren Zeitraum hinweg gespeichert wurden.

Die Fig. 3 veranschaulicht unterschiedliche Module, mit denen die die Auswerte- und Steuereinrichtung 24 betreffenden Verfahrensschritte durch geführt werden können. Neben den zur Fig. 1 beschriebenen Variante, in der eine Auswerte-und Steuereinrichtung 24 des Kraftfahrzeugs 10 das erfindungsgemäße Verfahren durchführen kann, kann das Verfahren alternativ durch eine Auswerte-und Steuereinrichtung 24 des mobilen Endgeräts 32 oder einer Auswerte-und Steuereinrichtung 24 einer kraftfahrzeugexternen Servervorrichtung 36 durchgeführt werden. In dem jeweiligen Modul, also in dem jeweiligen Gerät, kann eine durch das erfindungsgemäße Verfahren beschriebene Datenfusion stattfinden.

Insgesamt veranschaulichen die Ausführungsbeispiele, wie durch das erfindungsgemäße Verfahren eine Fahrerzustandsüberwachung ermöglicht wird, insbesondere in Hinblick auf pilotierte Fahrphasen, in denen es wichtig ist, die Bereitschaft zum Beispiel eines Fahrers zu monitoren, die Fahraufgabe wieder zu übernehmen.

Gemäß einem weiteren Ausführungsbeispiel kann zum Beispiel im Vorfeld eine Identifikation der Person in einem Kraftfahrzeugsitz 14 über eine geeignete Methode erfolgen, zum Beispiel über einen Fahrzeugschlüssel und/oder ein Smartphone und/oder ein Wearable. Mit anderen Worten kann die Identität des Benutzers 12 festgestellt werden (S4). Dies ist wichtig für eine eindeutige Zuordnung der Daten zu einem bestimmten Benutzer 12, um zum Beispiel über lernende Algorithmen "Normalzustände" und davon abweichende Zustände wie Stress oder Ermüdung zu identifizieren.

Dies erfolgt durch den Einsatz von zum Beispiel zwei geeigneten Sensoren der Sensoreinrichtung 22, die vorzugsweise in identischer Weise - also gleichzeitig und durch gleiche oder ähnliche Technologie - eine Bewegung, vorzugsweise die Atembewegung, Erschütterung und Beschleunigung messen. Ein erster Sensor kann sich dabei zum Beispiel in Brusthöhe einem Sicherheitsgurt 20 befinden, und ein Kontrollsensor kann ein einer beliebigen geeigneten Stelle im Kraftfahrzeugsitz 14 angebracht sein, an der er nicht von Bewegungen der des Benutzers 12 beeinflusst werden kann.

Eine Atemfrequenz und/oder eine Atemtiefe kann nun wie folgt erfasst werden: die beiden beispielhaften Sensoren können ihre Messergebnisse an die Auswerte-und Steuereinrichtung 24 senden, beispielsweise an eine Empfängereinheit der Auswerte-und Steuereinrichtung 24, welche die Daten auswerten kann. Der erste Sensor kann dabei sowohl die vom Fahrzeug erzeugten Bewegungsdaten und/oder Erschütterungsdaten und/oder Beschleunigungsdaten erfassen (S1), als auch die vom Menschen erzeugten Bewegungen.

Der Kontrollsponsor kann nur die vom Kraftfahrzeug 10 erzeugten Bewegung Daten und/oder Erschütterungsdaten und/oder Beschleunigungsdaten Erfassen (S2).

Ein Abgleich der von dem ersten Sensor und dem Kontrollsensor erfassten Daten kann beispielsweise in der Empfängereinheit erfolgen. Zum Abgleich ergeben sich die rein vom Menschen erzeugten Daten, aus denen Atemfrequenz und/oder Tiefe ermittelt werden (S3).

Die Auswerte-und Steuereinrichtung 24, beispielsweise mittels der Empfängereinheit, kann die Daten zu Atemfrequenz und/oder Atemtiefe wiederum an zum Beispiel ein Steuergerät/oder Softwaremodul der Auswerte-und Steuereinrichtung 24 geben, dass mithilfe lernende Algorithmen einen Abgleich zwischen normaler Atemfrequenz und/oder Atemtiefe des Benutzers 12 und gegebenenfalls abweichenden Zuständen wie zum Beispiel Stress (schnellere und flachere Atmung) und Ermüdung (langsamere, tiefere Atmung) geben.

In den beispielhaften Steuergerät können die Daten zu Atemfrequenz und/oder Atemtiefe optional mit anderen Sensorinformationen aus Fahrzeugsensoren und/oder zusätzlich aus einem tragbaren Accessoire 34, das von dem Benutzer 12 getragen wird, zu einem Fahrerzustandsindex verarbeitet werden (S7), der dann wiederum an die relevanten Kraftfahrzeugsysteme 28 (Komfort und/oder Sicherheit) gehen kann.

## Patentansprüche

1. Verfahren zum Betreiben eines Kraftfahrzeugs (10), das Verfahren aufweisend die Schritte:
- durch eine Sensoreinrichtung (22) des Kraftfahrzeugs (10) Erfassen mindestens eines eine Oberkörperbewegung eines Benutzers (12) des Kraftfahrzeugs (10) beschreibenden Atmungsbewegungsparameters, wobei ein Sensor der Sensoreinrichtung (22) zum Erfassen des Atmungsbewegungsparameters an einem Sicherheitsgurt (20) des Kraftfahrzeugs angeordnet (10) ist, und Erzeugen eines den erfassten Atmungsbewegungsparameter beschreibenden Atmungsbewegungssignals (S1), und
- Erfassen eines eine Bewegung eines Kraftfahrzeugsitzes (14) des Kraftfahrzeugs beschreibenden Fahrbewegungsparameters und Erzeugen eines den erfassten Fahrbewegungsparameter beschreibenden Fahrbewegungssignals (S2), und
- anhand des Atmungsbewegungssignals und des Fahrbewegungssignals Ermitteln eines Vitalparameters durch Normalisieren eines durch das Atmungsbewegungssignal beschriebenen Werts anhand eines Wertes des Fahrbewegungssignals, wobei der Vitalparameter eine Atmung des Benutzers (12) beschreibt, durch eine Auswerte- und Steuereinrichtung (24) (S3),
**gekennzeichnet durch** die durch die Auswerte- und Steuereinrichtung (24) durchgeführten Schritte:
- Feststellen eines Referenzwertebereiches, in dem ein ermittelter Wert des Vitalparameters liegt (S6), wobei der Referenzwertebereich in einer Speichereinrichtung (30) gespeicherte, benutzerspezifische Referenzwerte des Vitalparameters umfasst,
- anhand des festgestellten Referenzwertebereichs Feststellen eines Fahrerzustandsindex, der einen aktuellen physiologischen Zustand des Benutzers beschreibt (S7),
- in Abhängigkeit von dem ermittelten Fahrerzustandsindex Erzeugen eines Steuersignals zum Betreiben einer Fahrerassistenzeinrichtung (26) des Kraftfahrzeugs (10, S8), und
- Übertragen des Steuersignals an die Fahrerassistenzeinrichtung (26, S9).

2. Verfahren nach Anspruch 1, **gekennzeichnet durch** die durch die Auswerte- und Steuereinrichtung (24) durchgeführten Schritte:
- Feststellen einer Identität des Benutzers (12, S4),
- in Abhängigkeit von der festgestellten Identität Bereitstellen der benutzerspezifischen Referenzwerte des Vitalparameters (S5) und Feststellen des Referenzwertebereichs (S6).

3. Verfahren nach einem der vorhergehenden Ansprüche, **gekennzeichnet durch** den durch die Auswerte- und Steuereinrichtung (24) durchgeführten Schritt:
- Empfangen mindestens eines Vitalparametersignals, das einen Wert des Vitalparameters oder eines weiteren Vitalparameters beschreibt, vorzugsweise aus einem tragbaren Accessoire (34) und/oder aus einem mobilen Endgerät (32, S10),
wobei das Feststellen des Fahrerzustandsindex (S7) in Abhängigkeit von dem empfangenen Vitalparametersignals erfolgt.

4. Verfahren nach einem der vorhergehenden Ansprüche, **gekennzeichnet durch** den durch die Auswerte- und Steuereinrichtung (24) durchgeführten Schritt:
- Empfangen mindestens eines Betriebssignals aus einem Kraftfahrzeugsystem (28), das einen Betriebszustand des Kraftfahrzeugsystems (28) beschreibt (S10),
wobei das Feststellen des Fahrerzustandsindex (S7) in Abhängigkeit von dem empfangenen Betriebssignal erfolgt.

5. Verfahren nach einem der vorhergehenden Ansprüche, **gekennzeichnet durch** die durch die Auswerte- und Steuereinrichtung (24) durchgeführten Schritte:
- Speichern des ermittelten Werts des Vitalparameters als Referenzwert in der Speichereinrichtung (30, S11),
- Anpassen des Referenzwertbereichs in Abhängigkeit von dem gespeicherten Wert.

6. Auswerte- und Steuereinrichtung (24), vorzugsweise aufweisend einen Mikrokontroller (25) und/oder einen Mikroprozessor (25), wobei die Auswerte- und Steuereinrichtung (24) dazu eingerichtet ist, die eine Auswerte- und Steuereinrichtung (24) betreffenden Verfahrensschritte gemäß einem Verfahren nach einem der Ansprüche 1 bis 5 durchzuführen.

7. Kraftfahrzeug (10), aufweisend eine Fahrerassistenzeinrichtung (26) und eine Sensoreinrichtung (22), wobei die Sensoreinrichtung (22) einen Sensor zum Erfassen eines eine Oberkörperbewegung eines Benutzers (12) des Kraftfahrzeugs (10) beschreibenden Atmungsbewegungsparameters aufweist und einen Sensor zum Erfassen eines eine Bewegung eines Kraftfahrzeugsitzes (14) beschreibenden Fahrbewegungsparameters, das Kraftfahrzeug (10) **gekennzeichnet durch** eine Auswerte- und Steuereinrichtung (24) nach Anspruch 6, vorzugsweise wobei der Sensor zum Erfassen des Atmungsbewegungsparameters an einem Sicherheitsgurt (20) des Kraftfahrzeugs angeordnet ist, und wobei der Sensor zum Erfassen des Fahrbewegungsparameters an einem Kraftfahrzeugsitz (14) angeordnet ist.

8. Speichereinrichtung (30) mit einem Programmcode, der dazu eingerichtet ist, bei Ausführen durch eine Auswerte- und Steuereinrichtung (24) eines mobilen Endgeräts (32) ein Verfahren nach einem der Ansprüche 1 bis 5 durchzuführen.

9. Mobiles, portables Endgerät (32) mit einer Speichereinrichtung (30) nach Anspruch 8.

10. Servervorrichtung (36) zum Betreiben im Internet, aufweisend eine Speichereinrichtung (30) nach Anspruch 8.

## Claims

1. Method for operating a motor vehicle (10), with the method having the following steps:
- Detection, by a sensor device (22) of the motor vehicle (10), of at least one breathing movement parameter which describes an upper body movement of a user (12) of the motor vehicle (10), wherein a sensor of the sensor device (22) is positioned (10) on a seatbelt (20) of the vehicle to detect the breathing movement parameter and to generate a breathing movement signal (S1) which describes the detected breathing movement parameter, and
- Detection of a driving movement parameter which describes a movement of a motor vehicle seat (14) of the motor vehicle and generating of a driving movement signal (S2) which describes the detected driving movement parameter, and
- Using the breathing movement signal and the driving movement signal, identification of a vitality parameter by normalising a value described by the breathing movement signal using a value of the driving movement signal, wherein the vitality parameter describes a breathing movement of the user (12), by an evaluation and control device (24) (S3), ,
**characterised by** the steps carried out by the evaluation and control device (24):
- Ascertaining of a reference value range, in which an ascertained value of the vitality parameter lies (S6), wherein the reference value range comprises user-specific reference values of the vitality parameter stored in a storage device (30),
- Using the ascertained reference value range, ascertaining of a driver state index which describes (S7) a current physiological state of the user,
- Depending on the basis of the identified driver state index, generating of a control signal for operating a driver assistance device (26) of the motor vehicle (10, S8), and
- Transmission of the control signal to the driver assistance device (26, S9).

2. Method according to Claim 1, **characterised by** the steps carried out by the evaluation and control device (24):
- Ascertaining an identity of the user (12, S4),
- Providing the user-specific reference values of the vitality parameter (S5) and ascertaining the reference value range (S6), all on the basis of the ascertained identity.

3. Method according to any of the preceding claims, **characterised by** the step carried out by the evaluation and control device (24):
- Receiving at least one vitality parameter signal which describes a value of the vitality parameter or a further vitality parameter, preferably from a portable accessory (34) and/or a mobile terminal device (32, S10),
wherein the driver state index (S7) is ascertained on the basis of the vitality parameter signal received.

4. Method according to any of the preceding claims, **characterised by** the step carried out by the evaluation and control device (24):
- Receiving at least one operating signal from a motor vehicle system (28) which describes (S10) an operating state of the motor vehicle system (28),
wherein the driver state index (S7) is ascertained on the basis of the operating signal received.

5. Method according to any of the preceding claims, **characterised by** the steps carried out by the evaluation and control device (24):
- Storing the identified value of the vitality parameter as a reference value in the storage device (30, S11),
- Adjusting the reference value range on the basis of the stored value.

6. Evaluation and control device (24), preferably having a microcontroller (25) and/or a microprocessor (25), wherein the evaluation and control device (24) is configured so as to carry out the process steps relating to an evaluation and control device (24) in accordance with a method according to any of claims 1 to 5.

7. Motor vehicle (10) having a driver assistance device (26) and a sensor device (22), wherein the sensor device (22) has a sensor for detecting a breathing movement parameter which describes an upper body movement of a user (12) of the motor vehicle (10) and a sensor for detecting a driving movement parameter which describes a movement of a motor vehicle seat (14), the motor vehicle (10) being **characterised by** an evaluation and control device (24) according to Claim 6, preferably wherein the sensor for detecting the breathing movement parameter is positioned on a seatbelt (20) of the motor vehicle and wherein the sensor for detecting the driving movement parameter is positioned on a motor vehicle seat (14).

8. Storage device (30) with a program code configured so as to carry out a method according to any one of claims 1 to 5 when it is operated by an evaluation and control device (24) of a mobile terminal device (32).

9. Mobile portable terminal device (32) with a storage device (30) according to Claim 8.

10. Sever device (36) for operating via the Internet, having a storage device (30) according to Claim 8.

## Revendications

1. Procédé de fonctionnement d'un véhicule automobile (10), le procédé présentant les étapes consistant à :
- grâce à un dispositif capteur (22) du véhicule automobile (10), détecter au moins un paramètre de mouvement respiratoire décrivant un mouvement du haut du corps d'un utilisateur (12) du véhicule automobile (10), dans lequel un capteur du dispositif capteur (22) permettant de détecter le paramètre de mouvement respiratoire est agencé (10) au niveau d'une ceinture de sécurité (20) du véhicule automobile, et générer un signal de mouvement respiratoire (S1) décrivant le paramètre de mouvement respiratoire détecté, et
- détecter un paramètre de mouvement de déplacement décrivant un mouvement d'un siège de véhicule automobile (14) du véhicule automobile, et générer un signal de mouvement de déplacement (S2) décrivant le paramètre de mouvement de déplacement détecté, et
- à l'aide du signal de mouvement respiratoire et du signal de mouvement de déplacement, déterminer un paramètre vital grâce à une normalisation d'une valeur décrite par le signal de mouvement respiratoire en s'aidant d'une valeur du signal de mouvement de déplacement, dans lequel le paramètre vital décrit une respiration de l'utilisateur (12) grâce à un dispositif d'évaluation et de commande (24) (S3).
**caractérisé par** les étapes mises en oeuvre par le dispositif d'évaluation et de commande (24) et consistant à :
- déterminer une plage de valeurs de référence au sein de laquelle se trouve une valeur déterminée du paramètre vital (S6), dans lequel la plage de valeurs de référence comprend des valeurs de référence du paramètre vital spécifiques à l'utilisateur et mémorisées dans un dispositif de mémoire (30),
- à l'aide de la plage de valeurs de référence déterminée, déterminer un indice de condition de conducteur qui décrit une condition physiologique actuelle de l'utilisateur (S7),
- en fonction de l'indice de condition de conducteur déterminé, générer un signal de commande afin de faire fonctionner un dispositif d'assistance au conducteur (26) du véhicule automobile (10, S8), et
- transmettre le signal de commande au dispositif d'assistance au conducteur (26, S9).

2. Procédé selon la revendication 1, **caractérisé par** les étapes mises en oeuvre grâce au dispositif d'évaluation et de commande (24) et consistant à :
- déterminer une identité de l'utilisateur (12, S4),
- en fonction de l'identité déterminée, fournir les valeurs de référence du paramètre vital (S5) spécifiques à l'utilisateur et déterminer la plage de valeurs de référence (S6).

3. Procédé selon l'une quelconque des revendications précédentes, **caractérisé par** l'étape mise en oeuvre grâce au dispositif d'évaluation et de commande (24) et consistant à :
- recevoir au moins un signal de paramètre vital décrivant une valeur du paramètre vital ou d'un autre paramètre vital, de manière préférée à partir d'un accessoire portable (34) et/ou d'un dispositif terminal mobile (32, S10),
dans lequel la détermination de l'indice de condition de conducteur (S7) se fait en fonction du signal de paramètre vital reçu.

4. Procédé selon l'une quelconque des revendications précédentes, **caractérisé par** l'étape mise en oeuvre grâce au dispositif d'évaluation et de commande (24) et consistant à :
- recevoir d'un système de véhicule automobile (28) au moins un signal de fonctionnement qui décrit (S10) un état de fonctionnement du système de véhicule automobile (28),
dans lequel la détermination de l'indice de condition de conducteur (S7) se fait en fonction du signal de fonctionnement reçu.

5. Procédé selon l'une quelconque des revendications précédentes, **caractérisé par** les étapes mises en oeuvre par le dispositif d'évaluation et de commande (24) et consistant à :
- mémoriser la valeur déterminée du paramètre vital en tant que valeur de référence dans le dispositif de mémoire (30, S11),
- adapter la plage de valeurs de référence en fonction de la valeur mémorisée.

6. Dispositif d'évaluation et de commande (24), présentant de manière préférée un microcontrôleur (25) et/ou un microprocesseur (25), dans lequel le dispositif d'évaluation et de commande (24) est conçu pour mettre en oeuvre, conformément à un procédé selon l'une quelconque des revendications 1 à 5, les étapes de procédé concernant un dispositif d'évaluation et de commande (24).

7. Véhicule automobile (10), présentant un dispositif d'assistance au conducteur (26) et un dispositif capteur (22), dans lequel le dispositif capteur (22) présente un capteur permettant de détecter un paramètre de mouvement respiratoire décrivant un mouvement du haut du corps d'un utilisateur (12) du véhicule automobile (10) et un capteur permettant de détecter un paramètre de mouvement de déplacement décrivant un mouvement d'un siège de véhicule automobile (14), le véhicule automobile (10) étant **caractérisé par** un dispositif d'évaluation et de commande (24) selon la revendication 6, de manière préférée dans lequel le capteur permettant de détecter le paramètre de mouvement respiratoire est agencé au niveau d'une ceinture de sécurité (20) du véhicule automobile, et dans lequel le capteur permettant de détecter le paramètre de mouvement de déplacement est agencé au niveau d'un siège de véhicule automobile (14).

8. Dispositif de mémoire (30) avec un code de programme conçu pour mettre en oeuvre un procédé selon l'une quelconque des revendications 1 à 5 lorsqu'il est exécuté par un dispositif d'évaluation et de commande (24) d'un dispositif terminal mobile (32).

9. Dispositif terminal portable mobile (32) avec un dispositif de mémoire (30) selon la revendication 8.

10. Dispositif serveur (36) permettant un fonctionnement au sein d'Internet, présentant un dispositif de mémoire (30) selon la revendication 8.
